(19) **Europäisches Patentamt**

**European Patent Office**

**Office européen des brevets**

(11) **EP 0 528 846 B1**

(12) # EUROPEAN PATENT SPECIFICATION

(45) Date of publication and mention
of the grant of the patent:
**26.06.1996 Bulletin 1996/26**

(21) Application number: **91908594.4**

(22) Date of filing: **22.04.1991**

(51) Int. Cl.$^6$: **A61K 31/43**, A61K 9/16,
A61K 9/46

(86) International application number:
**PCT/GB91/00637**

(87) International publication number:
**WO 91/16893 (14.11.1991 Gazette 1991/26)**

(54) **PHARMACEUTICAL FORMULATION**

ARZNEIMITTEL

FORMULATION PHARMACEUTIQUE

(84) Designated Contracting States:
**AT BE CH DE DK ES FR GB GR IT LI LU NL SE**

(30) Priority: **27.04.1990 GB 9009473**

(43) Date of publication of application:
**03.03.1993 Bulletin 1993/09**

(73) Proprietor: **BEECHAM GROUP PLC
Brentford Middlesex TW8 9EP (GB)**

(72) Inventors:
• **GRIMMETT, Francis, W.
SmithKline Beecham
West Sussex BN14 8QH (GB)**

• **DAVIDSON, Nigel, Philip
SmithKline Beecham
West Sussex BN14 8QH (GB)**

(74) Representative: **Walker, Ralph Francis, Dr. et al
SmithKline Beecham plc
Corporate Intellectual Property
SB House
Great West Road
Brentford, Middlesex TW8 9BD (GB)**

(56) References cited:
**EP-A- 0 396 335          GB-A- 1 300 998
US-A- 4 888 177**

Printed by Rank Xerox (UK) Business Services
2.12.4/3.4

## Description

This invention relates to pharmaceutical compositions for oral administration of antibiotics and other medicaments with unpleasant taste characteristics, and particularly to compositions formulated for dispersion in water prior to administration.

Pharmaceutical compositions for oral administration of antibiotics and other medicaments are known. For example GB 1300998 discloses an effervescent granule formulation using soluble antibiotics. US 4888177 discloses a pharmaceutical formulation in which a core of carrier material is enveloped by a surrounding layer. EP 0396335A (prior art within the meaning of Art 54(3) EPC)discloses a chewable tablet formulation that includes an effervescent couple.

From the point of view of bioavailability, the preferred form of administration of sparingly soluble medicaments such as $\beta$-lactam antibiotics is often an aqueous suspension. However, there are problems associated with this form of administration. For example, such preparations in multidose form may have a limited shelf life, and usual methods of dose measurement lack accuracy.

Single dose powders for reconstitution in sachet form offer the advantages of suspensions without the problems of instability and measuring inaccuracy. Unfortunately, in the case of $\beta$-lactam antibiotics the problem of the unpleasant taste of these medicaments remains with such powder formulations.

Accordingly the present invention provides a pharmaceutical formulation, provided as a granular product containing a sparingly water soluble medicament and an effervescent couple which comprises a basic ingredient and an acidic ingredient distributed uniformly through the granule, the basic ingredient liberating carbon dioxide when it and the acidic ingredient are contacted with water, for dispersal in water to produce a suspension of the medicament which can be swallowed by a patient.

It has been found that the inclusion of an effervescent couple in pharmaceutical granules can enable very rapid formation (e.g. 10-30 seconds) of a suspension in a small volume of water for a relatively low fill weight presentation, in comparison to traditional sachet presentations of $\beta$-lactam antibiotics, without recourse to the use of additional disintegrating agents. The resulting suspension is found to be highly palatable and can consequently easily be swallowed by a patient.

Such sachet presentations are thus well-accepted by patients, especially small children and other groups of patients who may otherwise find the medicine difficult to take and who might otherwise refuse treatment. In addition there are commercial advantages of such a presentation. For example, a low weight product reduces raw material costs, enables more unit doses per batch and simplifies the manufacturing and packaging processes. The granular product of the invention also has good flow characteristics which result in improved sachet filling performance, ensuring that sachets may be well sealed.

Preferred medicaments are $\beta$-lactam antibiotics such as penicillins and cephalosporins, especially amoxycillin and ampicillin, preferably amoxycillin trihydrate. A preferred $\beta$-lactamase inhibitor is clavulanic acid, preferably as potassium clavulanate. Typically, the ratio of antibiotic to inhibitor is 4:1 or 2:1 by weight, but ratios of 12:1 to 1:1 may be used. The weight of the antibiotic in a unit dose may range from 125mg to 3g, expressed in terms of the activity of the antibiotic. The weight of the antibiotic in the formulation, calculated as the free acid, may range from 5% to 50% preferably 40% to 50% based on the weight of the formulation.

The effervescent couple typically comprises citric acid or sodium hydrogen citrate and sodium bicarbonate, but other physiologically acceptable alkaline or alkaline earth metal carbonate mixtures may be used, for example tartaric, adipic, fumaric or malic acids, and sodium, potassium or calcium bicarbonates or sodium glycine carbonate.

The weight of the acidic ingredient may be in the range of 0.5% to 20%, e.g. 1.0% to 10%, preferably 1.5% to 5%, of the weight of the formulation.

The weight of the basic component may be in the range 0.5% to 30%, e.g. 1.0 to 20%, preferably 1.5% to 10%, of the weight of the mixture.

The granular formulation may contain any of the conventional excipients such as diluents/fillers/bulking agents used in pharmaceutical products, for example lactose, fructose, mannitol or sorbitol alone or in combination e.g. making up 0.1% to 60%, typically 30% to 50% by weight of the formulation. Other conventional excipients may include lubricants e.g. making up 0.1% to 3%, typically 0.25% to 2.5% by weight of the formulation such as magnesium stearate, sweetening agents such as sugars sodium saccharin and aspartame e.g. making up 0.1% to 2% by weight and flavouring agents e.g. lemon and/or lime typically making up 0 to 20% by weight of the formulation. Multidose products for reconstitution may include suspending agents such as Xanthan gum e.g. Keltrol - Trade Mark, (a sodium, potassium or calcium salt of a partially acetylated polysaccharide containing D glucose, D mannose and D glucoronic acid units). Alternative thickeners are hydroxypropyl methyl celluloses or hydroxypropyl cellulose (e.g. Klucel - Trade Mark), sodium carboxymethyl - cellulose - carmellose or aerosil. Typically such suspending agents may be present at 1% to 5% by weight. Multidose products may also include preservatives such as sodium benzoate, typically at 0.2% to 1.5% by weight. A desiccant such as syloid (Trade Mark) may also be included for moisture sensitive antibiotics.

The invention also provides a process for the preparation of such a pharmaceutical formulation, comprising admixing the medicament and the effervescent couple, and subsequently compacting the mixture into a granular product.

A preferred size fraction for the granular formulation of the invention is less than 1000µ, e.g. 30µ to 600µ, particularly 100µ to 425µ. To prepare the granular formulation of the invention the starting components, especially the medicament are preferably finely milled to a particle size of less than 200µ, to result in a particle size of typically <30µ-200µ. Starting materials of <30µ-1000µ (with the exception of the medicament) may be used. The coarse excipient fraction may improve flow and hence will aid the compaction process.

Typically the manufacturing process involves the following stages:

(a) Mill the medicament finely at fast speed, through a 0.020 inch (0.5mm) screen.

(b) Mill the diluent/filler/bulking agent and sweeteners at slow speed through an 0.040 inch (1.0mm) screen.

(c) Sieve through a 20 mesh screen the flavours, effervescent couple components and magnesium stearate.

(d) Blend the components and compress the mix on a tabletting or slugging machine to give compacts (slugs) of medium density or roller compact the mix to a medium pressure and product density.

(e) Pass the compacted material through a mill at low speed fitted with a screen, to form granules.

(f) Sieve the granules and collect the desired size fraction. Recycle the coarser and finer material to the compaction equipment if it is desired to limit the size range of the granule.

(g) Fill the granule under low humidity cover.

Preferably the entire process (a) to (g) is carried out under a low humidity, e.g. <30% RH and a low temperature e.g. 10-25°C. Preferably the components used are anhydrous or substantially anhydrous.

The granular formulation may be coated with an acid soluble polymer to assist rapid and widespread release of the medicament to occur in the stomach, or they may be coated with an enteric (acid resistant polymer) to assist rapid release in the intestine. Suitable acid soluble polymers include Eudragit E (Trade Mark) - a cationic polymer synthesised from dimethylaminoethyl methacrylate, ethycellulose or ethylcellulose mixtures with water soluble polymers such as hydroxypropyl cellulose or hydroxypropylmethyl cellulose.

The granules are preferably packed in conventional unit dose sachets, e.g. composed of a laminate of polymer, paper and foil. A common granular formulation may be used at a range of different fill weights to provide a range of unit doses e.g. 125 mg, 250 mg, 500 mg, 1g and 3g of amoxycillin. Alternatively the granular formulation may be provided in jars etc. as a multidose presentation for make up in water.

The invention also provides a process for the use of a pharmaceutical formulation as described above in the manufacture of a medicament for the treatment of bacterial infections.

The invention also provides a pharmaceutical formulation as described above for use in the treatment of bacterial infections.

The invention is illustrated by the following Example.

EP 0 528 846 B1

Example 1

| 250mg. Dose Fizzy Granule (β-lactam antibiotic) | | |
|---|---|---|
| Ingredients | mg/sachet | (% w/w) |
| Amoxycillin Trihydrate equivalent to Amoxycillin free acid | 250.00 | 41.667 |
| Magnesium stearate | 6.75 | 1.125 |
| Citric acid | 12.50 | 2.083 |
| Sodium bicarbonate | 25.50 | 4.167 |
| Sodium saccharin | 2.50 | 0.417 |
| Lemon dry flavour | 27.50 | 4.583 |
| Lime dry flavour | 1.38 | 0.230 |
| Sorbitol B.P. | 90.00 | 15.000 |
| Mannitol U.S.P. | 184.37 | 30.72 |
| Total | 600 mg | 100.00% |
| (all of the above ingredients were used in a substantially anhydrous state). | | |

Manufacturing Procedure for Example 1

The amoxycillin was first milled finely at a high speed through an 0.02 inch (0.5mm) screen. Then the mannitol and sodium saccharin were milled at a slow speed through an 0.04 inch (1mm) screen. The flavours, citric acid, sodium bicarbonate and magnesium stearate were then sieved through a 20 mesh sieve. The mixture was compressed on a rotary tabletting machine to give compacts of density 0.39-0.40.

The compacted material was then milled at low speed, 1750 rpm, with knives forward and fitted with an 0.097 inch (0.25mm) screen. The granules were sieved on a 20 mesh overlying an 80 mesh screen and the 20 - 80 fraction was collected. This granular product could then be filled into containers or sachets, or tabletted in a conventional manner. The procedure was performed in a humidity of 30% RH or less, at 10-25°C.

Alternative fruity or citrus flavours could be used in the formulation of example 1. Furthermore the quoted percentages could be varied by ± 10% without any significant effect on the properties of the formulation.

The quantities of ingredients quoted in example 1 are for a 250mg unit dose amoxycillin formulation. By simply increasing or decreasing the quoted quantities in direct proportion formulations for unit doses of other weights of amoxycillin can be made up.

**Claims**

**Claims for the following Contracting States : AT, BE, CH, DE, DK, FR, GB, IT, LI, LU, NL, SE**

1. A pharmaceutical formulation, provided as a granular product containing a sparingly water soluble medicament and an effervescent couple which comprises a basic ingredient and an acidic ingredient distributed uniformly through the granule, the basic ingredient liberating carbon dioxide when it and the acidic ingredient are contacted with water, for dispersal in water to produce a suspension of the medicament which can be swallowed by a patient.

2. A formulation according to claim 1 characterised in that the medicament is a β-lactam antibiotic.

3. A formulation according to claim 2 characterised in that the antibiotic is amoxycillin.

4. A formulation according to claim 2 or 3 characterised by containing 5% to 50% by weight of the antibiotic.

4

5. A formulation according to claim 4 characterised by containing 40% to 50% by weight of the antibiotic.

6. A formulation according to any one of preceding claims 1 to 5 characterised in that the effervescent couple comprises citric acid or sodium hydrogen citrate and sodium bicarbonate.

7. A formulation according to any one of preceding claims 1 to 5 characterised in that the acidic ingredient is selected from tartaric, adipic, fumaric or malic acid, and the basic ingredient is selected from sodium, potassium or calcium bicarbonate or sodium glycine carbonate.

8. A formulation according to any one of preceding claims 1 to 7 characterised in that the acidic ingredient is present in the range 0.5% to 20% of the weight of the formulation.

9. A formulation according to claim 8 characterised in that the acidic ingredient is present in the range 1% to 10% of the weight of the formulation.

10. A formulation according to any one of preceding claims 1 to 9 characterised in that the basic ingredient is present in the range 0.5% to 30% of the weight of the formulation.

11. A formulation according to any one of preceding claims 1 to 10 wherein the size of the granular product is in the range 30 to 600μ.

12. A formulation according to claim 11 characterised in that the size of the granular product is in the range 100 to 425μ.

13. A formulation according to any one of preceding claims 1 to 12 characterised by a composition within ± 10% of the following:

| | |
|---|---|
| Amoxycillin trihydrate (equivalent to amoxycillin free acid) | 41.667 wt % |
| Magnesium stearate | 1.125 wt % |
| Citric acid | 2.083 wt % |
| Sodium bicarbonate | 4.167 wt % |
| Sodium saccharin | 0.417 wt % |
| Lemon dry flavour | 4.583 wt % |
| Lime dry flavour | 0.230 wt % |
| Sorbitol BP | 15.000 wt % |
| Mannitol USP | 30.720 wt % |

14. A process for the preparation of a pharmaceutical formulation as claimed in any one of preceding claims 1 to 13 comprising admixing a medicament and an effervescent couple and subsequently compacting the mixture into a granular product.

15. The use of a pharmaceutical formulation as claimed in any one of preceding claims 1 to 14 for the manufacture of a medicament for the treatment of bacterial infections.

16. A pharmaceutical formulation as claimed in any one of preceding claims 1 to 14 for use in the treatment of bacterial infections.

**Claims for the following Contracting States : ES, GR**

1. A process for the preparation of a pharmaceutical formulation provided as a granular product containing a sparingly water soluble medicament and an effervescent couple which comprises a basic ingredient and an acidic ingredient distributed uniformly through the granule, the basic ingredient liberating carbon dioxide when it and the acidic ingredient are contacted with water, for dispersal in water to produce a suspension of the medicament which can be swallowed by a patient.

2. A process according to claim 1 characterised in that the medicament is a β-lactam antibiotic.

3. A process according to claim 2 characterised in that the antibiotic is amoxycillin.

4. A process according to claim 2 or 3 characterised in that the formulation contains 5% to 50% by weight of the antibiotic.

5. A process according to claim 4 characterised in that the formulation contains 40% to 50% by weight of the antibiotic.

6. A process according to any one of preceding claims 1 to 5 characterised in that the effervescent couple comprises citric acid or sodium hydrogen citrate and sodium bicarbonate.

7. A process according to any one of preceding claims 1 to 5 characterised in that the acidic ingredient is selected from tartaric, adipic, fumaric or malic acid, and the basic ingredient is selected from sodium, potassium or calcium bicarbonate or sodium glycine carbonate.

8. A process according to any one of preceding claims 1 to 7 characterised in that the acidic ingredient is present in the range 0.5% to 20% of the weight of the formulation.

9. A process according to claim 8 characterised in that the acidic ingredient is present in the range 1% to 10% of the weight of the formulation.

10. A process according to any one of preceding claims 1 to 9 characterised in that the basic ingredient is present in the range 0.5% to 30% of the weight of the formulation.

11. A process according to any one of preceding claims 1 to 10 wherein the size of the granular product is in the range 30 to 600μ.

12. A process according to claim 11 characterised in that the size of the granular product is in the range 100 to 425μ.

13. A process according to any one of preceding claims 1 to 12 characterised in that the composition is within ± 10% of the following:

| | |
|---|---|
| Amoxycillin trihydrate (equivalent to amoxycillin free acid) | 41.667 wt % |
| Magnesium stearate | 1.125 wt % |
| Citric acid | 2.083 wt % |
| Sodium bicarbonate | 4.167 wt % |
| Sodium saccharin | 0.417 wt % |
| Lemon dry flavour | 4.583 wt % |
| Lime dry flavour | 0.230 wt % |
| Sorbitol BP | 15.000 wt % |
| Mannitol USP | 30.720 wt % |

14. A process according to any one of preceding claims 1 to 13 comprising admixing a medicament and an effervescent couple and subsequently compacting the mixture into a granular product.

15. The use of a pharmaceutical formulation as claimed in any one of preceding claims 1 to 14 in a process for the manufacture of a medicament for the treatment of bacterial infections.

**Patentansprüche**

**Patentansprüche für folgende Vertragsstaaten : AT, BE, CH, DE, DK, FR, GB, IT, LI, LU, NL, SE**

1. Pharmazeutische Zubereitung in Form eines Granulatprodukts, enthaltend ein mäßig wasserlösliches Medikament und ein Brausepaar, welches einen basischen Bestandteil und einen sauren Bestandteil umfaßt, die im Granulatkorn gleichmäßig verteilt sind, wobei der basische Bestandteil, wenn er und der saure Bestandteil mit Wasser in Kontakt kommen, Kohlendioxid freisetzt zur Dispersion in Wasser, wodurch eine Suspension des Medikaments hergestellt wird, die von einem Patienten geschluckt werden kann.

2. Zubereitung nach Anspruch 1, dadurch gekennzeichnet, daß das Medikament ein β-Lactam-Antibiotikum ist.

3. Zubereitung nach Anspruch 2, dadurch gekennzeichnet, daß das Antibiotikum Amoxycillin ist.

4. Zubereitung nach Anspruch 2 oder 3, dadurch gekennzeichnet, daß sie 5 bis 50 Gew.-% des Antibiotikums enthält.

5. Zubereitung nach Anspruch 4, dadurch gekennzeichnet, daß sie 40 bis 50 Gew.-% des Antibiotikums enthält.

6. Zubereitung nach einem der vorstehenden Ansprüche 1 bis 5, dadurch gekennzeichnet, daß das Brausepaar Zitronensäure oder Natriumhydrogencitrat sowie Natriumhydrogencarbonat umfaßt.

7. Zubereitung nach einem der vorstehenden Ansprüche 1 bis 5, dadurch gekennzeichnet, daß der saure Bestandteil aus Wein-, Adipin-, Fumar- oder Äpfelsäure gewählt wird, und der basische Bestandteil aus Natrium-, Kalium- oder Calciumhydrogencarbonat oder Natriumglycincarbonat gewählt wird.

8. Zubereitung nach einem der vorstehenden Ansprüche 1 bis 7, dadurch gekennzeichnet, daß der saure Bestandteil in einer Menge von 0,5 bis 20 Gew.-% der Zubereitung vorhanden ist.

9. Zubereitung nach Anspruch 8, dadurch gekennzeichnet, daß der saure Bestandteil in einer Menge von 1 bis 10 Gew.-% der Zubereitung vorhanden ist.

10. Zubereitung nach einem der vorstehenden Ansprüche 1 bis 9, dadurch gekennzeichnet, daß der basische Bestandteil in einer Menge von 0,5 bis 30 Gew.-% der Zubereitung vorhanden ist.

11. Zubereitung nach einem der vorstehenden Ansprüche 1 bis 10, wobei die Größe des Granulatprodukts im Bereich von 30 bis 600 µm liegt.

12. Zubereitung nach Anspruch 11, dadurch gekennzeichnet, daß die Größe des Granulatprodukts im Bereich von 100 bis 425 µm liegt.

13. Zubereitung nach einem der vorstehenden Ansprüche 1 bis 12, gekennzeichnet durch eine Zusammensetzung innerhalb 10% der folgenden Mengenangaben:

| | |
|---|---|
| Amoxycillin-trihydrat (äquivalent zur freien Säure Amoxycillin) | 41,667 Gew.-% |
| Magnesiumstearat | 1,125 Gew.-% |
| Zitronensäure | 2,083 Gew.-% |
| Natriumhydrogencarbonat | 4,167 Gew.-% |
| Natriumsaccharin | 0,417 Gew.-% |
| Zitronentrockengeschmack | 4,583 Gew.-% |
| Limettentrockengeschmack | 0,230 Gew.-% |
| Sorbit BP | 15,000 Gew.-% |
| Mannit USP | 30,720 Gew.-% |

14. Verfahren zur Herstellung einer pharmazeutischen Zubereitung nach einem der vorstehenden Ansprüche 1 bis 13, umfassend Mischen eines Medikaments sowie eines Brausepaars und nachfolgendes Pressen des Gemischs zu einem Granalutprodukt.

15. Verwendung einer pharmazeutischen Zubereitung nach einem der vorstehenden Ansprüche 1 bis 14 zur Herstellung eines Medikaments zur Behandlung von bakteriellen Infektionen.

16. Pharmazeutische Zubereitung nach einem der vorstehenden Ansprüche 1 bis 14 zur Verwendung bei der Behandlung von bakteriellen Infektionen.

**Patentansprüche für folgende Vertragsstaaten : ES, GR**

1. Verfahren zur Herstellung einer pharmazeutischen Zubereitung, in Form eines Granulatprodukts enthaltend ein mäßig wasserlösliches Medikament und ein Brausepaar enthält, wobei das Brausepaar einen basischen Bestandteil und einen saure Bestandteil umfaßt, die im Granulatkorn gleichmäßig verteilt sind, wobei der basische Bestandteil, wenn er und der saure Bestandteil mit Wasser in Kontakt kommen, Kohlendioxid freisetzt zur Dispersion in Wasser, wodurch eine Suspension des Medikaments hergestellt wird, die von einem Patienten geschluckt werden kann.

2. Verfahren nach Anspruch 1, dadurch gekennzeichnet, daß das Medikament ein β-Lactam-Antibiotikum ist.

3. Verfahren nach Anspruch 2, dadurch gekennzeichnet, daß das Antibiotikum Amoxycillin ist.

4. Verfahren nach Anspruch 2 oder 3, dadurch gekennzeichnet, daß die Zubereitung 5 bis 50 Gew.-% des Antibiotikums enthält.

5. Verfahren nach Anspruch 4, dadurch gekennzeichnet, daß die Zubereitung 40 bis 50 Gew.-% des Antibiotikums enthält.

6. Verfahren nach einem der vorstehenden Ansprüche 1 bis 5, dadurch gekennzeichnet, daß das Brausepaar Zitronensäure oder Natriumhydrogencitrat sowie Natriumhydrogencarbonat umfaßt.

7. Verfahren nah einem der vorstehenden Ansprüche 1 bis 5, dadurch gekennzeichnet, daß der saure Bestandteil aus Wein-, Adipin-, Fumar- oder Äpfelsäure gewählt wird, und der basische Bestandteil aus Natrium-, Kalium- oder Calciumhydrogencarbonat oder Natriumglycincarbonat gewählt wird.

8. Verfahren nach einem der vorstehenden Ansprüche 1 bis 7, dadurch gekennzeichnet, daß der saure Bestandteil in einer Menge von 0,5 bis 20 Gew.-% der Zubereitung vorhanden ist.

**9.** Verfahren nach Anspruch 8, dadurch gekennzeichnet, daß der saure Bestandteil in einer Menge von 1 bis 10 Gew.-% du Zubereitung vorhanden ist.

**10.** Verfahren nach einem der vorstehenden Ansprüche 1 bis 9, dadurch gekennzeichnet, daß der basische Bestandteil in einer Menge von 0,5 bis 30 Gew.-% der Zubereitung vorhanden ist.

**11.** Verfahren nach einem der vorstehenden Ansprüche 1 bis 10, wobei die Größe des Granulatprodukts im Bereich von 30 bis 600 μm liegt.

**12.** Verfahren nach Anspruch 11, dadurch gekennzeichnet, daß die Größe des Granulatprodukts im Bereich von 100 bis 425 μm liegt.

**13.** Verfahren nach einem der vorstehenden Ansprüche 1 bis 12, gekennzeichnet durch eine Zusammensetzung innerhalb 10% der folgenden Mengenangaben:

| | |
|---|---|
| Amoxycillin-trihydrat (äquivalent zur freien Säure Amoxycillin) | 41,667 Gew.-% |
| Magnesiumstearat | 1,125 Gew.-% |
| Zitronensäure | 2,083 Gew.-% |
| Natriumhydrogencarbonat | 4,167 Gew.-% |
| Natriumsaccharin | 0,417 Gew.-% |
| Zitronentrockengeschmack | 4,583 Gew.-% |
| Limettentrockengeschmack | 0,230 Gew.-% |
| Sorbit BP | 15,000 Gew.-% |
| Mannit USP | 30,720 Gew.-% |

**14.** Verfahren nach einem der vorstehenden Ansprüche 1 bis 13, umfassend Mischen eines Medikaments sowie eines Brausepaars und nachfolgendes Pressen des Gemisches zu einem Granalutprodukt.

**15.** Verwendung einer pharmazeutischen Zubereitung nach einem der vorstehenden Ansprüche 1 bis 14 in einem Verfahren zur Herstellung eines Medikaments zur Behandlung von bakteriellen Infektionen.

**Revendications**

**Revendications pour les Etats contractants suivants : AT, BE, CH, DE, DK, FR, GB, IT, LI, LU, NL, SE**

**1.** Formulation pharmaceutique, présentée sous forme d'un produit granulaire contenant un médicament faiblement hydrosoluble et un couple effervescent qui comprend un ingrédient basique et un ingrédient acide répartis uniformément dans la totalité du granule, l'ingrédient basique libérant de l'anhydride carbonique lorsque cet ingrédient et l'ingrédient acide sont mis en contact avec de l'eau, à des fins de dispersion dans l'eau pour produire une suspension du médicament qui peut être avalée par un patient.

**2.** Formulation suivant la revendication 1, caractérisée en ce que le médicament consiste en un antibiotique faisant partie des β-lactames.

**3.** Formulation suivant la revendication 2, caractérisée en ce que l'antibiotique consiste en amoxycilline.

**4.** Formulation suivant la revendication 2 ou 3, caractérisée en ce qu'elle contient 5 % à 50 % en poids de l'antibiotique.

**5.** Formulation suivant la revendication 4, caractérisée en ce qu'elle contient 40 % à 50 % en poids d'antibiotique.

6. Formulation suivant l'une quelconque des revendications 1 à 5 précédentes, caractérisée en ce que le couple effervescent comprend de l'acide citrique ou du citrate acide de sodium et du bicarbonate de sodium.

7. Formulation suivant l'une quelconque des revendications 1 à 5 précédentes, caractérisée en ce que l'ingrédient acide est choisi entre l'acide tartrique, l'acide adipique, l'acide fumarique et l'acide malique, et l'ingrédient basique est choisi entre le bicarbonate de sodium, de potassium ou de calcium et le carbonate de glycine sodique.

8. Formulation suivant l'une quelconque des revendications 1 à 7 précédentes, caractérisée en ce que l'ingrédient acide est présent en une quantité comprise dans l'intervalle de 0,5 % à 20 % du poids de la formulation.

9. Formulation suivant la revendication 8, caractérisée en ce que l'ingrédient acide est présent en une quantité comprise dans l'intervalle de 1 % à 10 % du poids de la formulation.

10. Formulation suivant l'une quelconque des revendications 1 à 9 précédentes, caractérisée en ce que l'ingrédient basique est présent en une quantité comprise dans l'intervalle de 0,5 % à 30 % du poids de la formulation.

11. Formulation suivant l'une quelconque des revendications 1 à 10 précédentes, dans laquelle les dimensions des granules du produit sont comprises dans l'intervalle de 30 à 600 $\mu$m.

12. Formulation suivant la revendication 11, caractérisée en ce que les dimensions des granules du produit sont comprises dans l'intervalle de 100 à 425 $\mu$m.

13. Formulation suivant l'une quelconque des revendications 1 à 12 précédentes, caractérisée par une composition des constituants suivants, dans les limites de ±10 % :

| | |
|---|---|
| trihydrate d'amoxycilline (équivalent à l'amoxycilline sous forme d'acide libre) | 41,667 % en poids |
| stéarate de magnésium | 1,125 % en poids |
| acide citrique | 2,083 % en poids |
| bicarbonate de sodium | 4,167 % en poids |
| saccharine sodique | 0,417 % en poids |
| arôme sec de citron | 4,583 % en poids |
| arôme sec de limette | 0,230 % en poids |
| sorbitol pharmacopée britannique | 15,000 % en poids |
| mannitol pharmacopée des Etats-Unis d'Amérique | 30,720 % en poids. |

14. Procédé de préparation d'une formulation pharmaceutique suivant l'une quelconque des revendications 1 à 13 précédentes, comprenant le mélange d'un médicament et d'un couple effervescent, puis le compactage du mélange sous forme d'un produit granulaire.

15. Utilisation d'une formulation pharmaceutique suivant l'une quelconque des revendications 1 à 14 précédentes pour la production d'un médicament destiné au traitement d'infections bactériennes.

16. Formulation pharmaceutique suivant l'une quelconque des revendications 1 à 14 précédentes, destinée à être utilisée dans le traitement d'infections bactériennes.

**Revendications pour les Etats contractants suivants : ES, GR**

1. Procédé de préparation d'une formulation pharmaceutique présentée sous forme d'un produit granulaire contenant un médicament faiblement hydrosoluble et un couple effervescent qui comprend un ingrédient basique et un ingrédient acide répartis uniformément dans la totalité du granule, l'ingrédient basique libérant de l'anhydride carbonique lorsque cet ingrédient et l'ingrédient acide sont mis en contact avec de l'eau, pour une dispersion dans de l'eau afin de produire une suspension du médicament qui peut être avalée par un patient.

2. Procédé suivant la revendication 1, caractérisé en ce que le médicament consiste en un antibiotique faisant partie des β-lactames.

3. Procédé suivant la revendication 2, caractérisé en ce que l'antibiotique consiste en amoxycilline.

4. Procédé suivant la revendication 2 ou 3, caractérisé en ce que la formulation contient 5 % à 50 % en poids de l'antibiotique.

5. Procédé suivant la revendication 4, caractérisé en ce que la formulation contient 40 % à 50 % en poids de l'antibiotique.

6. Procédé suivant l'une quelconque des revendications 1 à 5 précédentes, caractérisé en ce que le couple effervescent comprend de l'acide citrique ou du citrate acide de sodium et du bicarbonate de sodium.

7. Procédé suivant l'une quelconque des revendications 1 à 5 précédentes, caractérisé en ce que l'ingrédient acide est choisi entre l'acide tartrique, l'acide adipique, l'acide fumarique et l'acide malique, et l'ingrédient basique est choisi entre le bicarbonate de sodium, de potassium ou de calcium et le carbonate de glycine sodique.

8. Procédé suivant l'une quelconque des revendications 1 à 7 précédentes, caractérisé en ce que l'ingrédient acide est présent en une quantité comprise dans l'intervalle de 0,5 % à 20 % du poids de la formulation.

9. Procédé suivant la revendication 8, caractérisé en ce que l'ingrédient acide est présent en une quantité comprise dans l'intervalle de 1 % à 10 % du poids de la formulation.

10. Procédé suivant l'une quelconque des revendications 1 à 9 précédentes, caractérisé en ce que l'ingrédient basique est présent en une quantité comprise dans l'intervalle de 0,5 % à 30 % du poids de la formulation.

11. Procédé suivant l'une quelconque des revendications 1 à 10 précédentes, dans lequel les dimensions des granules du produit sont comprises dans l'intervalle de 30 à 600 μm.

12. Procédé suivant la revendication 11, caractérisé en ce que les dimensions des granules du produit sont comprises dans l'intervalle de 100 à 425 μm.

13. Procédé suivant l'une quelconque des revendications 1 à 12 précédentes, caractérisé en ce que la composition comprend les constituants suivants, dans les limites de ±10 % :

| trihydrate d'amoxycilline (équivalent à l'amoxycilline sous forme d'acide libre) | 41,667 % en poids |
|---|---|
| stéarate de magnésium | 1,125 % en poids |
| acide citrique | 2,083 % en poids |
| bicarbonate de sodium | 4,167 % en poids |
| saccharine sodique | 0,417 % en poids |
| arôme sec de citron | 4,583 % en poids |
| arôme sec de limette | 0,230 % en poids |
| sorbitol pharmacopée britannique | 15,000 % en poids |
| mannitol pharmacopée des Etats-Unis d'Amérique | 30,720 % en poids. |

14. Procédé suivant l'une quelconque des revendications 1 à 13 précédentes, comprenant le mélange d'un médicament et d'un couple effervescent, puis le compactage du mélange sous forme d'un produit granulaire.

15. Utilisation d'une formulation pharmaceutique suivant l'une quelconque des revendications 1 à 14 précédentes dans un procédé de production d'un médicament pour le traitement d'infections bactériennes.